(19)

Europäisches
Patentamt
European
Patent Office
Office européen
des brevets

(11) **EP 2 308 469 A1**

(12) **EUROPEAN PATENT APPLICATION**

(43) Date of publication:
**13.04.2011 Bulletin 2011/15**

(51) Int Cl.:
***A61K 9/12*** *(2006.01)*      ***A61K 47/12*** *(2006.01)*
***A61K 47/02*** *(2006.01)*

(21) Application number: **10014830.3**

(22) Date of filing: **18.05.2006**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK YU**

(30) Priority: **18.05.2005 US 682356 P**

(62) Document number(s) of the earlier application(s) in
accordance with Art. 76 EPC:
**06770654.9 / 1 906 922**

(71) Applicant: **Pulmatrix, Inc.
Lexington MA 02421 (US)**

(72) Inventors:
• **Watanabe, Wiwik S
Union City
California 94587 (US)**

• **Thomas, Matthew
Quincy
Massachusetts 02169 (US)**
• **Katstra, Jeffrey
Watertown
Massachusetts 02472 (US)**
• **Clarke, Robert
Canton
Massachusetts 02021 (US)**

(74) Representative: **O'Connell, Maura et al
FRKelly
27 Clyde Road
Ballsbridge
Dublin 4 (IE)**

Remarks:
This application was filed on 22-11-2010 as a
divisional application to the application mentioned
under INID code 62.

(54) **Formulations for alteration of biophysical properties of mucosal lining**

(57)      Conductive formulations containing conductive agents, such as salts, ionic surfactants, or other substances that are in an ionized state or easily ionized in an aqueous or organic solvent environment, and methods of use, have been developed. When applied to mucosal lining fluids, the formulation alters the physical properties such as the surface tension, surface elasticity, and bulk viscosity of the mucosal lining. The formulation is administered in an amount sufficient to alter biophysical properties in the mucosal linings of the body.

**EP 2 308 469 A1**

**Description**

**Cross-Reference to Related Applications**

This application claims priority to U.S.S.N. 60/682,356, entitled *"Formulations for Alteration of Biophysical Properties of Mucosal Lining",* filed May 18, 2005.

**Field of the Invention**

The present invention is in the field of formulations and methods to control particle shedding from mucosal surfaces, and alter uptake kinetics of drug molecules and pathogens.

**Background of the Invention**

Many organs have a liquid mucosal lining whose biophysical properties can facilitate or impede normal function. A wide array of adverse health effects have been associated with the properties of a mucosal lining, for instance, particles 'shed' from the upper airway mucosal lining fluid (UAL) during normal exhalation may carry viable, infectious bacterial or viral pathogens, such as Severe Acute Respiratory Syndrome ("SARS"), influenza, tuberculosis, capable of spreading to healthy individuals through inhalation; the surface tension of the UAL has been shown to play a role in obstructive sleep apnea syndrome; and alteration of the mucosal lining of the intestinal tract by viruses/mycobacteria may lead to inflammatory bowel disease over time. Controlled alteration of the mucosal lining's biophysical properties can effectively treat/prevent many of these adverse health effects. Additionally, the biophysical properties of the mucosal lining can affect the uptake of pathogens and drug molecules into the body and therefore manipulation of these properties may be used to block pathogen uptake or improve drug molecule uptake/bioavailability.

Airborne transmission is one of the main routes of pathogen infection. Aerosols composed of mucus droplets originating in the lungs and nasal cavities are produced when a human or animal coughs or simply breathes. These bioaerosols can contain pathogens that transmit the disease upon inhalation by exposed humans or animals. In addition, respirable pathogenic bioaerosols produced in the upper airways can be re-breathed by the host leading to parenchymal infection with exacerbated disease outcomes.

Viral and bacterial infections are frequently highly contagious, especially when spread by respiration. The reports regarding SARS, known to be caused by a corona virus, are proof of how rapidly an infection can spread when it is transmitted through air contact. Other diseases such as influenza are spread by air contact, and rapidly reach epidemic proportions, with high numbers of fatalities in elderly and immunocompromised populations.

Epidemics of respiratory infections are not limited to humans. Foot-and-mouth disease virus (FMDV) is the etiologic agent of foot-and-mouth disease (FMD), which is a disease of cattle, swine, and other cloven-footed animals. FMD is characterized by the formation of vesicles on the tongue, nose, muzzle, and coronary bands of infected animals. Several unique characteristics make the virus one of the most economically devastating diseases in the world today. The ease with which it may be transmitted by contact and aerosol, combined with its enhanced ability to initiate infections, virtually ensures that most, if not all, animals in a herd will contract FMD. The long-term survival of FMDV in infected animals' tissues and organs, especially when refrigerated, creates an opportunity for its national and international transmission through the food chain. Multiple serotypes and numerous subtypes reduce the effectiveness and reliability of vaccines. The possible development of carriers in vaccinated animals and those that have recovered from FMD provides additional potential sources of new outbreaks. These features create a disease that can have a major economic impact on livestock operations around the world. The FMD epidemic in British livestock remains an ongoing cause for concern, with new cases still arising in previously unaffected areas (Ferguson, et al., Nature 2001 414(6861):329). The parameter estimates obtained in a dynamic model of disease spreading show that extended culling programs were essential for controlling the epidemic to the extent achieved, but demonstrate that the epidemic could have been substantially reduced in scale had the most efficient methods been used earlier in the outbreak.

In addition to reducing or preventing the 'shedding' of contagious aerosol agents from the UAL, alteration of the biophysical properties of mucosal linings within the body can achieve several other ends. One is the control over the occurrence of obstructive sleep apnea (OSA). OSA occurs during sleep and refers to frequent closure and re-opening of the upper airway. OSA is caused by sleep-relaxed throat muscles allowing the throat to constrict completely during inhalation and is especially prevalent in people with narrow throats (either hereditary or due to external factors such as swelling, fat deposits, etc.). Certain allergy medicines such as antihistamines as well as alcohol consumption can also relax the throat leading to OSA. Chronic OSA is observed in approximately 4% of the general population and leads to frequent waking and an increased work-of-breathing throughout the night which can cause fatigue and sudden sleeping spells during normal activities. A study on anesthetized rabbits demonstrated that the surface tension of the UAL influenced the patency of the rabbit's upper airway, and surmised that the surface properties of the UAL likely played a role in the occurrence of OSA (Kirkness, J. P. et al. J Physiol 2003, 547.2 pp. 603-611).

Another region of the body where the mucosal lining plays an important role is in the gastrointestinal system. Irritable Bowel Syndrome (IBS) is inflammation of the intestines and can develop for several different reasons (although the exact cause remains unknown). In many cases the development of IBS appears to be a genetic susceptibility to the disease, however recent studies have suggested that certain viruses or bacteria can alter the mucosal lining of the intestines and that over time this alteration can lead to the development of IBS (A.D.A.M., http://adam.about.com/reports/ 000069_1.htm, May 12, 2005). The studies have reported a link between the development of IBS in children and an increased frequency of childhood infections, such as measles which has been specifically identified. Other infectious agents capable of altering the properties of the mucosal lining that are under investigation are *Escherichia Coli* (E. Coli) and *Cytomegalovirus* (CMV). One study of IBS reported that over 43% of those enrolled were infected with CMV.

The mucosal lining is the first screening mechanism of the immune system in many areas of the body, selectively allowing beneficial components through to the underlying epithelial layer and from there into the bloodstream and preventing the uptake of harmful pathogens and allergens. The primary component of mucus that protects against harmful uptake is secretory immunoglobulin A (IgA), which exhibits antiviral, antibacterial, anti-inflammatory and antiallergenic activity (Williams, J. E., Alternative Medicine Review Vol. 8, Number 9, 2003). This chemically inhibits pathogens/allergens from passing through the mucosal layer, trapping them at or near the surface where they are eventually flushed through the system and expelled in the stool, urine or, in the respiratory system, through the action of ciliated cells. However, many pathogens have developed sophisticated chemical transport systems to penetrate the mucosal layer, and in immunocompromised individuals the normal mechanisms for preventing pathogen uptake make be impaired or disabled. Altering the biophysical properties of the mucosal lining (for example, increasing gelation at the mucus surface) could reduce or prevent the ability of the pathogen to physically penetrate the mucosal lining. A side-effect of the mucosal lining immune response is that beneficial drugs may be taken to be harmful by the body and blocked from passing through the mucosal lining. Alteration of the biophysical properties of the mucosal lining may increase a drug molecule's permeability through the lining and improve uptake of drug molecules. There have been various methods/formulations introduced to effectively deliver poorly absorbable antibiotics and peptides through mucosal membranes. Ionic surfactants, such as sodium lauryl sulfate and chelating agents such as ethylene diamine tetraacetic (EDTA), have been found to enhance intestinal absorption of such molecules. Unfortunately, large amount of these substances have been found to be harmful to the mucosal membrane.

WO03/092654 to David Edwards et al. describes a method for diminishing the spread of inhaled infections by delivering materials such as surfactants that suppress bioaerosol expiration. This technique works on the basis of altering the surface or other physical properties of the endogenous surfactant fluid in the lungs, and thereby favoring fewer exhaled bioaerosol particles.

WO 2005/084638 to Pulmatrix et al. describes a non-surfactant solution that, via dilution of endogenous surfactant fluid, alters physical properties such as surface tension, surface elasticity and bulk viscosity of lung mucus lining fluid. The aerosolized material may be an isotonic saline solution, a hypertonic saline solution or other solution containing osmotically active materials. The formulation may be administered as a powder where the particles consist essentially of a salt or osmotically-active substance that dilutes endogenous surfactant fluid. The aerosol may be a solution, suspension, spray, mist, vapor, droplets, particles, or a dry powder. Typical concentrations of salts or sugars are in the range of up to 5 or 6% solute. The formulation is administered in an effective amount to decrease surface instabilities in the liquid lining the airways of the lung, without causing expectoration.

It would be desirable to have additional means of limiting bioaerosol formation and/or spread of infection.

It is therefore an object of the present invention to provide methods for altering the biophysical properties of the mucosal linings present within the body.

It is a further object of the present invention to provide compositions for altering the biophysical properties of the mucosal linings present within the body.


Summary of the Invention


Conductive formulations containing conductive agents, such as salts, ionic surfactants, or other substances that are in an ionized state or easily ionized in an aqueous or organic solvent environment, and methods of use, have been developed. One or more active agents, such as antivirals, antimicrobials, anti-inflammatories, proteins or peptides, may optionally be included with the formulation. The active agent may be administered with or incorporated into the formulation, or may be administered after the conductive formulation is administered. When applied to mucosal lining fluids, the formulation alters the physical properties such as the gel characteristics at the air/liquid interface, surface tension, surface viscosity, surface elasticity, and bulk viscoelasticity of the mucosal lining. The formulation is administered in an amount sufficient to alter biophysical properties in the mucosal linings of the body. The formulations may be administered for several different purposes: reducing the spreading of infectious diseases, both viral and bacterial, such as SARS, influenza, tuberculosis, and RSV in humans and hoof and mouth disease in cloven-footed animals; relieving airway irritation and congestion due to respiratory conditions including acute infection (e.g. common cold), asthma, chronic bronchitis, emphysema, bronchiectasis; minimizing ambient contamination due to particle formation during breathing, coughing, sneezing, or talking which is particularly important in the clean room applications; decreasing or preventing the occurrence of obstructive sleep apnea and some cases of irritable bowel syndrome; and controlling the uptake kinetics of drug molecules and pathogens.

**Brief Description of the Drawings**

Figure 1 is a schematic of the simulated respiratory machine (SRM) apparatus used in the Examples.

Figure 2 is a bar graph of the cumulative particle counts 300 nm) following administration of different aerosolized formulations on the mucus mimetic layer, as measured *in vitro* using the SRM apparatus (3 psi air pressure, 2 mm mimetic height (6.4 mL total mimetic volume), 15 minutes cross-linking time, and 2 minutes formulation aerosolization time).

Figure 3 is a bar graph of cumulative particle counts 300 nm) following administration of different aerosolized formulations in water and in saline) on the mucus mimetic layer, as measured *in vitro* using the SRM apparatus (in water and in saline) (3 psi air pressure, 2 mm mimetic height (6.4 mL total mimetic volume), 2 minutes formulation aerosolization time and 15 minutes cross-linking time).

Figure 4 is a graph of formulation conductivity ($\mu$S/cm) as measured by a zetasizer, ZetaPALS (Brookhaven Instruments Corp, Holtsville, NY) versus the cumulative particle counts (> 300 nm) as measured *in vitro* using the SRM apparatus.

Figure 5 is a graph of surface loss tangent ("Tan $\delta$") as measured by Interfacial Surface Rheometer versus the cumulative particle counts 300 nm) as measured *in vitro* using the SRM apparatus.

Figure 6A is a bar graph of the cumulative particle counts following administration of different aerosolized formulations on the mucus mimetic layer, as *measured in vitro* using the SRM apparatus (4 psi air pressure, 2 mm mimetic height (6.4 mL total mimetic volume), 2 minutes formulation aerosolization time and 15 minutes cross-linking time). Figure 6B is a bar graph of the percent suppression of particle counts as compared to the untreated mucus mimetic for the same formulations under the same conditions as depicted in Figure 6A.

**Detailed Description of the Invention**

A wide array of adverse health effects are associated with the properties of a mucosal lining. Accordingly, the ability to alter the biophysical properties of the mucosal lining is a or treatment or prevention of the spread of disease. Additionally, the alteration of the biophysical properties for the mucosal lining can be used to control the uptake of drugs as well as pathogens.

The mucosal lining consists of complex substances. The cross-linking mechanism occurring in the mucosal lining can be altered by altering the charge and charge distribution on the surface of the lining. This subsequently causes biophysical alteration of the mucosal lining that is beneficial for prevention and treatment of various diseases; suppression of bioaerosol formation during breathing/talking/coughing/sneezing (thus minimizes spreading of airborne respiratory infectious diseases, such as influenza, SARS, etc); relieving airway irritation and congestion due to respiratory conditions including acute infection (e.g. common cold), asthma, chronic bronchitis, emphysema, bronchiectasis; increased uptake/ bioavailability of drug molecules; and blockage of the pathogen uptake through the mucosal lining.

Biophysical properties, such as the viscoelasticity and bulk viscosity, for the mucosal lining can be determined using *in vitro* experiments. The viscoelastic modulus, G*, is written as a complex number involving the real, elastic (storage) modulus, G', and the imaginary, viscous (loss) modulus, G". This relationship can be expressed as

$$G^* = G' + i G'' \text{ or } |G^*| = \sqrt{(G')^2 + (G'')^2} \quad (\text{Eq. 1})$$

where G* is the viscoelastic modulus, also known as the mechanical impedance,
G' is the elastic modulus or storage modulus, and
G" is the viscous modulus or loss modulus.

The tangent of the phase lag (written as $\delta$) between the applied force (stress) and the measured response (strain) is equal to the ratio of the viscous modulus to the elastic modulus. This is commonly referred to as "the loss tangent" or "Tan $\delta$", and can be expressed as follows:

$$\text{Tan } \delta = G''/G' \qquad (\text{Eq. 2})$$

The loss tangent is used as a measure of the damping of the system. For a viscoelastic sample, $0° < \delta < 90°$. For a semi-liquid sample, $\delta\ 45°$ and $G''>G'$. For a semi-solid sample, $\delta < 45°$ and $G''$. For example, the greater Tan $\delta$, the greater $G''$ is relative to $G'$; and therefore the more viscous and less able to store energy the material. In a completely elastic solid, Tan $\delta$ equals zero (i.e. $G' \gg G''$). In order to measure the viscoelastic properties of a material, a sinusoidal stress is applied and a sinusoidal response (strain) develops (lagging the stress by some amount) (*see* Example 4). Both the magnitude of the response and the lag can be measured, and from these measurements $G'$ and $G''$ can be determined.

Lung mucociliary clearance is the primary mechanism by which the airways are kept clean from particles present in the liquid film that coats them. The conducting airways are lined with ciliated epithelium that beat to drive a layer of mucus towards the larynx, clearing the airways from the lowest ciliated region over the course of 24 hours. The fluid coating consists of water, sugars, proteins, glycoproteins, and lipids. It is generated in the airway epithelium and the submucosal glands, and the thickness of the layer ranges from several microns in the trachea to approximately 1 micron in the distal airways in humans, rats, and guinea pigs.

A second important mechanism for keeping the lungs clean is via momentum transfer from the air flowing through the lungs to the mucus coating. Coughing increases this momentum transfer and is used by the body to aid the removal of excess mucus. It becomes important when mucus cannot be adequately removed by ciliary beating alone, as occurs in mucus hypersecretion associated with many disease states. Air speeds as high as 200 m/s can be generated during a forceful cough. The onset of unstable sinusoidal disturbances at the mucus layer has been observed at such air speeds. This disturbance results in enhanced momentum transfer from the air to the mucus and consequently accelerates the rate of mucus clearance from the lungs. Experiments have shown that this disturbance is initiated when the air speed exceeds some critical value that is a function of film thickness, surface tension, and viscosity (M. Gad-El-Hak, R.F. Blackwelder, J.J. Riley. J. Fluid Mech. (1984) 140:257-280). Theoretical predictions and experiments with mucus-like films suggest that the critical speed to initiate wave disturbances in the lungs is in the range of 5-30 m/s.

Papineni and Rosenthal (J. Aerosol Med., 1997, 10(2): 105-116) have demonstrated that during standard mouth and nose breathing, or during coughing, normal human subjects expire tens to hundreds of liquid bioaerosol droplets, with a preponderance of exhaled bioaerosol droplets having a diameter smaller than one micron. Coughing was shown to give rise to the greatest number of particles, although the mean exhaled particle size remained significantly less than a micron. The majority of these particles are larger than most inhaled pathogens, i.e., greater than 150 nm. For instance, some common inhaled pathogens have characteristic sizes in this range: tuberculosis, 1,000-5,000 nm; influenza, 80-120 nm; measles, 100-250 nm; chicken pox, 120-200 nm; and FMD, 27-30 nm.

I. Formulations

The formulations described herein are effective to alter the biophysical properties of the mucosal lining. These properties include, for example, increasing gelation at the mucus surface, the surface tension of the mucosal lining, the surface elasticity of the mucosal lining, and the bulk viscoelasticity of the mucosal lining. Preferred formulations for altering the biophysical properties of the lung's lining fluid are formulations containing certain charge concentrations and mobility, and thus liquid conductivity.

Suitable formulations include aqueous solutions or suspensions that are conductive (also referred to herein as the "conductive formulation(s)"). Suitable conductive formulations typically have conductivity values of greater than 5,000 $\mu$S/cm, preferably greater than 10,000 $\mu$S/cm, and more preferably greater than 20,000 $\mu$S/cm. In a preferred embodiment, the formulation has a specific conductivity that is greater than the specific conductivity of isotonic saline. These formulations are particularly useful when administered to a patient to suppress particle exhalation. Solution conductivity is a product of the ionic strength, concentration, and mobility (the latter two contribute to the conductivity of the formulation as a whole). Any form of the ionic components (anionic, cationic, or zwitterionic) can be used. These conductive materials may alter the mucosal lining properties by acting, for example, as a cross-linking agent within the mucus. The ionic components in the formulations described herein may interact with the strongly linked anionic glycoproteins within normal tracheobronchial mucus. These interactions may influence the state of the air/liquid surface of the airway lining fluid and transiently the nature of the physical entanglements due to covalent and noncovalent interactions, including hydrogen bonding, hydrophobic, and electrostatic interactions (Dawson, M., et al., The Journal of Biological Chemistry. Vol. 278, No. 50, pp. 50393-50401 (2003)).

Optionally the formulation includes mucoactive or mucolytic agents, such as MUCSAC and MUC5B mucins, DNA, N-acetylcysteine (NAC), cysteine, nacystelyn, dornase alfa, gelsolin, heparin, heparin sulfate, P2Y2 agonists (e.g. UTP, INS365), and nedocromil sodium.

Formulations can be designed for specific application. In some embodiments, the formulation is administered to a mucosal surface to make the mucosal lining more liquid-like, while in others the formulation is administered to make the mucosal lining more solid-like. For example, for minimizing pathogen uptake or reducing particle exhalation, the formulation is designed to increase the solidity of the mucosal lining, i.e. where $\delta$ is less than 45° (*see* Eq. 2 above). In contrast, to increase drug uptake, the formulation is designed to increase the liquidity of the mucosal lining, i.e. where $\delta$ is greater than 45° (*see* Eq. 2 above).

a. Conductive agents

The formulations contain substances that are easily ionized in an aqueous or organic solvent environment (also referred to herein as "conductive agents"), such as salts, ionic surfactants, charged amino acids, charged proteins or peptides, or charged materials (cationic, anionic, or zwitterionic). Suitable salts include any salt form of the elements sodium, potassium, magnesium, calcium, aluminum, silicon, scandium, titanium, vanadium, chromium, cobalt, nickel, copper, manganese, zinc, tin, and similar elements. Examples include sodium chloride, sodium acetate, sodium bicarbonate, sodium carbonate, sodium sulfate, sodium stearate, sodium ascorbate, sodium benzoate, sodium biphosphate, sodium phosphate, sodium bisulfite, sodium citrate, sodium borate, , sodium gluconate, calcium chloride, calcium carbonate, calcium acetate, calcium phosphate, calcium alginite, calcium stearate, calcium sorbate, calcium sulfate, calcium gluconate, magnesium carbonate, magnesium sulfate, magnesium stearate, magnesium trisilicate, potassium bicarbonate, potassium chloride, potassium citrate, potassium borate, potassium bisulfite, potassium biphosphate, potassium alginate, potassium benzoate, magnesium chloride, cupric sulfate, chromium chloride, stannous chloride, and sodium metasilicate and similar salts. Suitable ionic surfactants include sodium dodecyl sulfate (SDS) (also known as sodium lauryl sulfate (SLS)), magnesium lauryl sulfate, Polysorbate 20, Polysorbate 80, and similar surfactants. Suitable charged amino acids include L-Lysine, L-Arginine, Histidine, Aspartate, Glutamate, Glycine, Cysteine, Tyrosine. Suitable charge proteins or peptides include proteins and peptides containing the charged amino acids, Calmodulin (CaM), and Troponin C. Charged phospholipids, such as 1,2-dioleoyl-sn-glycero-3-ethylphosphocholine triflate (EDOPC) and alkyl phosphocholine trimesters, can be used. Negatively charged phospholipids include phosphatidylinositol, phosphatidylserine, phosphatidylglycerol and phosphatidic acid, cardiolipins, dialkanoyl phosphatidyl glycerols (dipalmitoyl phosphatidyl glycerol and dimyristoyl phosphatidyl glycerol), phosphatidylinositol 4-phosphate (PIP), phosphatidylinositol 4,5-bisphosphate (PIP2), and phosphatidylethanolamines. Positively charged phospholipids include dioleoyl trimethylammonium propane, esters of phosphatidic acids, such as dipalmitoylphosphatidic acid and distearoyl-phosphatidic acid with aminoalcohols such as hydroxyethylenediamine.

The preferred formulations are formulations containing salts, such as saline (0.15 M NaCl or 0.9%) solution, $CaCl_2$ solution, $CaCl_2$ in saline solution, or saline solution containing ionic surfactants, such as SDS or SLS. In the preferred embodiment, the formulation contains saline solution and $CaCl_2$. Suitable concentration ranges of the salt or other conductive/charged compounds can vary from about 0.01 % to about 20% (weight of conductive or charged compound/total weight of formulation), preferably between 0.1 % to about 10% (weight of conductive or charged compound/total weight of formulation), most preferably between 0.1 to 7% (weight of conductive or charged compound/total weight of formulation). In a preferred embodiment, the formulation contains a hypertonic saline solution (*i.e.* sodium chloride concentration greater than 0.9% by weight).

Saline solutions have long been delivered chronically to the lungs with small amounts of therapeutically active agents, such as beta agonists, corticosteroids, or antibiotics. For example, VENTOLIN® Inhalation Solution (GSK) is an albuterol sulfate solution used in the chronic treatment of asthma and exercise-induced bronchospasm symptoms. A VENTOLIN® solution for nebulization is prepared (by the patient) by mixing 1.25-2.5mg of albuterol sulfate (in 0.25-0.5mL of aqueous solution) into sterile normal saline to achieve a total volume of 3mL. No adverse effects have been found to be associated with the delivery of saline to the lungs by VENTOLIN® nebulization, even though nebulization times can range from 5-15 minutes. Saline is also delivered in more significant amounts to induce expectoration. Often these saline solutions are hypertonic (sodium chloride concentrations greater than 0.9% by weight, often as high as 5% by weight) and generally they are delivered for up to 20 minutes.

b. Active Ingredients

The formulations disclosed herein can be used by any route for delivery of a variety of organic or inorganic molecules, especially small molecule drugs, such as antiviral and antibacterial drugs including antibiotics, antihistamines, bronchodilators, cough suppressants, anti-inflammatories, vaccines, adjuvants and expectorants. Examples of macromolecules include proteins and large peptides, polysaccharides and oligosaccharides, and DNA and RNA nucleic acid molecules and their analogs having therapeutic, prophylactic or diagnostic activities. Nucleic acid molecules include genes, antisense molecules that bind to complementary DNA to inhibit transcription, and ribozymes. Preferred agents are antiviral, steroid, bronchodilators, antibiotics, mucus production inhibitors, and vaccines.

In the preferred embodiment, the concentration of the active agent ranges from about 0.01 % to about 20% by weight. In a more preferred embodiment, the concentration of active agent ranges from between 0.1 % to about 10%.

II. Carriers and Aerosols for Administration

The formulation may be delivered in a solution, a suspension, a spray, a mist, a foam, a gel, a vapor, droplets, particles, or a dry powder form (for example, using a metered dose inhaler including HFA propellant, a metered dose inhaler with non-HFA propellant, a nebulizer, a pressurized can, or a continuous sprayer). Carriers can be divided into those for administration via solutions or suspensions (liquid formulations) and those for administration via particles (dry powder formulations).

A. Dosage forms for Administration to different mucosal surfaces

For administration to mucosal surfaces in the respiratory tract, the formulation is typically in the form of solution, suspension or dry powder. Preferably, the formulation is aerosolized. The formulation can be generated via any aerosol generators, such as dry powder inhaler (DPI), nebulizers or pressurized metered dose inhalers (pMDI). The term "aerosol" as used herein refers to any preparation of a fine mist of particles, typically less than 10 microns in diameter. The preferred mean diameter for aqueous formulation aerosol particles is about 5 microns, for example between 0.1 and 30 microns, more preferably between 0.5 and 20 microns and most preferably between 0.5 and 10 microns.

For administration to the oral mucosa, including buccal mucosa, the formulation may be administered as a solid that dissolves following administration to the mouth and/or adheres to the mucosal surface, or a liquid.

For administration to the vaginal mucosa, the formulation is preferably in the form of a viscous solution or suspension, gel, foam, ointment, creme, lotion, or suppository. Optionally, the formulation may be placed in a device for insertion, such as a vaginal ring.

For administration to the gastrointestinal mucosa, the formulation is typically in the form of solution, suspension, solid dosage form (e.g. capsule or tablet), or dry powder. Optionally, the formulation is bioadhesive, and may contain one or more bioadhesive polymers or other excipients.

B. Liquid Formulations

Aerosols for the delivery of therapeutic agents to the respiratory tract have been developed. See, for example, Adjei, A. and Garren, J. Pharm. Res., 7: 565-569 (1990); and Zanen, P. and Lamm, J.-W. J.Int. J. Pharm., 114: 111-115 (1995). These are typically formed by atomizing the solution or suspension under pressure through a nebulizer or through the use of a metered dose inhaler ("MDI"). In the preferred embodiment, these are aqueous solutions or suspensions.

**C. Dry Powder Formulations**

The geometry of the airways is a major barrier for drug dispersal within the lungs. The lungs are designed to entrap particles of foreign matter that are breathed in, such as dust There are three basic mechanisms of deposition: impaction, sedimentation, and Brownian motion (J.M. Padfield. 1987. In: D. Ganderton & T. Jones eds. Drug Delivery to the Respiratory Tract, Ellis Harwood, Chicherster, U.K.). Impaction occurs when particles are unable to stay within the air stream, particularly at airway branches. They are adsorbed onto the mucus layer covering bronchial walls and cleaned out by mucocilliary action. Impaction mostly occurs with particles over 5 $\mu$m in diameter. Smaller particles (<5 $\mu$m) can stay within the air stream and be transported deep into the lungs. Sedimentation often occurs in the lower respiratory system where airflow is slower. Very small particles (<0.6 $\mu$m) can deposit by Brownian motion. This regime is undesirable because deposition cannot be targeted to the alveoli (N. Worakul & J.R. Robinson. 2002. In: Polymeric Biomaterials, 2nd ed. S. Dumitriu ed. Marcel Dekker. New York).

The preferred mean diameter for aerodynamically light particles for inhalation is at least about 5 microns, for example between about 5 and 30 microns, most preferably between 3 and 7 microns in diameter. The particles may be fabricated with the appropriate material, surface roughness, diameter and tap density for localized delivery to selected regions of the respiratory tract such as the deep lung or upper airways. For example, higher density or larger particles may be used for upper airway delivery. Similarly, a mixture of different sized particles, provided with the same or different therapeutic agent may be administered to target different regions of the lung in one administration.

As used herein, the phrase "aerodynamically light particles" refers to particles having a mean or tap density less than about 0.4 g/cm$^3$. The tap density of particles of a dry powder may be obtained by the standard USP tap density measurement. Tap density is a standard measure of the envelope mass density. The envelope mass density of an isotropic particle is defined as the mass of the particle divided by the minimum sphere envelope volume in which it can be enclosed. Features contributing to low tap density include irregular surface texture and porous structure.

Dry powder formulations ("DPFs") with large particle size have improved flowability characteristics, such as less aggregation (Visser, J., Powder Technology 58: 1-10 (1989)), easier aerosolization, and potentially less phagocytosis. Rudt, S. and R. H. Muller, J. Controlled Release, 22: 263-272 (1992); Tabata, Y., and Y. Ikada, J. Biomed. Mater. Res., 22: 837-858 (1988). Dry powder aerosols for inhalation therapy are generally produced with mean diameters primarily in the range of less than 5 microns, although a preferred range is between one and ten microns in aerodynamic diameter. Ganderton, D., J. Biopharmaceutical Sciences, 3:101-105 (1992); Gonda, I. "Physico-Chemical Principles in Aerosol Delivery," in Topics in Pharmaceutical Sciences 1991, Crommelin, D. J. and K. K. Midha, Eds., Medpharm Scientific Publishers, Stuttgart, pp. 95-115 (1992). Large "carrier" particles (containing no drug) have been co-delivered with therapeutic aerosols to aid in achieving efficient aerosolization among other possible benefits. French, D. L., Edwards, D. A. and Niven, R. W., J. Aerosol Sci., 27: 769-783 (1996). Particles with degradation and release times ranging from seconds to months can be designed and fabricated by established methods in the art.

Particles can consist of the conductive agent(s), alone, or in combination with drug, antiviral, antibacterial, antimicrobial, surfactant, proteins, peptides, polymer, or combinations thereof. Representative surfactants include L-alpha-phosphatidylcholine dipalmitoyl ("DPPC"), diphosphatidyl glycerol (DPPG), 1,2-Dipalinitoyl-sn-glycero-3-phospho-L-serine (DPPS), 1,2-Dipalmitoyl-sn-glycero-3-phosphocholine (DSPC), 1,2-Distearoyl-sn-glycero-3-phosphoethanolamine (DSPE), 1-palmitoyl-2-oleoylphosphatidylcholine (POPC), fatty alcohols, polyoxyethylene-9-lauryl ether, surface active fatty acids, sorbitan trioleate (Span 85), glycocholate, surfactin, poloxomers, sorbitan fatty acid esters, tyloxapol, phospholipids, and alkylated sugars. Polymers may be tailored to optimize particle characteristics including: i) interactions between the agent to be delivered and the polymer to provide stabilization of the agent and retention of activity upon delivery; ii) rate of polymer degradation and thus drug release profile; iii) surface characteristics and targeting capabilities via chemical modification; and iv) particle porosity. Polymeric particles may be prepared using single and double emulsion solvent evaporation, spray drying, solvent extraction, solvent evaporation, phase separation, simple and complex coacervation, interfacial polymerization, and other methods well known to those of ordinary skill in the art. Particles may be made using methods for making microspheres or microcapsules known in the art. The preferred methods of manufacture are by spray drying and freeze drying, which entails using a solution containing the conductive/charge materials, spraying the solution onto a substrate to form droplets of the desired size, and removing the solvent.

### III. Uses for Conductive Formulations

Formulations capable of altering the biophysical properties of mucosal lining within the body have been developed for delivery by any available routes and can be used for several different purposes: reducing the spreading of infectious diseases (both viral and bacterial) such as SARS, influenza, tuberculosis, and RSV in humans and hoof and mouth disease in cloven-footed animals; relieving airway irritation and congestion due to respiratory conditions including acute infection (e.g. common cold), asthma, chronic bronchitis, emphysema, bronchiectasis; minimizing ambient contamination due to particle formation during breathing, coughing, sneezing, or talking (which is particularly important in the clean room applications); decreasing or preventing the occurrence of obstructive sleep apnea, some cases of irritable bowel syndrome, chronic obstructive pulmonary disease (COPD), cystic fibrosis, and dysentery; and controlling the uptake kinetics of drug molecules and pathogens.

### A. Administration of Drug Delivery Formulations

In one embodiment, the conductive formulation contains a drug and a suitable conductivity for increasing the viscoelasticity of the mucosal membrane at the site of administration of the formulation. The drug may be a conductive agent, or the formulation may contain a drug and a conductive agent. The conductive formulation may be administered via inhalation to interact with the air/liquid interface of tracheobronchial mucus layer to modify the biophysical properties of the mucus layer and enhance drug delivery and diffusion through to the airway cells. Alternatively the formulation may be administered parenterally, orally, rectally, vaginally, or topically, or by administration to the ocular space to interact with other mucosal surfaces.

In some cases it has been noted that high sputum viscoelasticity causes a more macroporous transport of nanoparticles (Sanders, N.N., De Smedt, S.C., Rompaey, E.V., Simoens, P., De Baets, F. & Demeester, J. (2000) Am J Respir Crit Care Med. 162,1905-1911.). Therefore, these formulations may cause a net driving force or gradient from the air/liquid interface towards the cells for increased drug uptake.

### B. Administration of Drug Delivery Pre-Treatment formulations

The conductive formulations may be used as a drug delivery "pre-treatment". In one embodiment, a conductive formulation is delivered, and then a drug formulation is delivered to the patient. The drug may be any of a wide range of drugs including anti-virals, siRNA formulations, and liposomal formulations. When the conductive formulation is administered via inhalation, the conductive formulation is designed to alter the interfacial biophysical properties for the airway lining fluid by increasing or decreasing their elasticity, depending on the desired result, to improve subsequent drug delivery. Alternatively the formulation may be administered parenterally, orally, rectally, vaginally, or topically, or by administration to the ocular space to interact with other mucosal surfaces. In some cases it has been noted that inducing large gradients in biophysical properties (surface tension and viscoelastic properties) improve transport or immersion depth of submicron Teflon particles (Im Hof, V., Gehr, P., Gerber, V., Lee, M.M. & Schurch, S. (1997) Respir. Physiol. 109, 81-93. and Schurch, S., Gehr, P., Im Hof, V., Geiser, M. & Green, F. (1990) Respir Physiol. 80, 17-32.).

## C. Administration of Conductive formulations to Alter

## Pathogen Transport and Uptake

In another embodiment, the conductive formulation contains a suitable conductivity for increasing the viscoelasticity or altering the charge gradient of the mucosal membrane at the site of administration of the formulation to prevent or reduce the uptake kinetics of pathogens in the body. In some cases it has been noted that inducing large charge gradients improves transport and adhesion of microbial agents (see Goldberg, S, et al., J Bacteriology 172, 5650-5654 (1990)) and can alter virus entry (Davis, H.E., et al., Biophys J, 86, 1234-1242 (2004)).

The conductive formulation is designed to alter the interfacial biophysical properties for the airway lining fluid by increasing or decreasing their elasticity, depending on the desired result, or alter the charge interaction at the airway lining fluid/airway tissue interface. The formulation may contain cationic or anionic molecules to achieve this effect. This change in charge gradient subsequently will prevent or slow uptake and transport of a pathogen intracellulary or alter release of replicated pathogen back into the extracellular space. Alternatively, the charge gradient may alter the adhesion and immunogenicity of a pathogen.

The conductive formulation is typically administered via inhalation. However, the formulation may also be administered parenterally, orally, rectally, vaginally, or topically, or by administration to the ocular space to interact with other mucosal surfaces.

## D. Administration of Conductive formulations to reduce amount of exhaled particles

In another embodiment, the conductive formulation contains a suitable conductivity for increasing the viscoelasticity of the mucosal membrane at the site of administration of the formulation to suppress or reduce the formation of bioaerosol particles formation during breathing, coughing, sneezing, and/or talking. Preferably, the formulation is administered to one or more individuals who have bacterial or viral infections to decrease or limit the spread of pulmonary infections to other animals or humans, especially viral or bacterial infections. Alternatively, the formulation may be administered to healthy individuals, or immunocompromised individuals to prevent or reduce the uptake of pathogens by the body.

## E. Administration to the Respiratory Tract

The respiratory tract is the structure involved in the exchange of gases between the atmosphere and the blood stream. The lungs are branching structures ultimately ending with the alveoli where the exchange of gases occurs. The alveolar surface area is the largest in the respiratory system and is where drug absorption occurs. The alveoli are covered by a thin epithelium without cilia or a mucus blanket and secrete surfactant phospholipids. J.S. Patton & R.M. Platz. 1992. Adv. Drug Del. Rev. 8:179-196

The respiratory tract encompasses the upper airways, including the oropharynx and larynx, followed by the lower airways, which include the trachea followed by bifurcations into the bronchi and bronchioli. The upper and lower airways are called the conducting airways. The terminal bronchioli then divide into respiratory bronchioli which lead to the ultimate respiratory zone, the alveoli or deep lung. The deep lung, or alveoli, is the primary target of inhaled therapeutic aerosols for systemic drug delivery.

The formulations are typically administered to an individual to deliver an effective amount to alter physical properties such as surface tension and viscosity of endogenous fluid in the upper airways, thereby enhancing delivery to the lungs and/or suppressing coughing and/or improving clearance from the lungs. Effectiveness can be measured using a system as described below. For example, saline can be administered in a volume of 1 gram to a normal adult. Exhalation of particles is then measured. Delivery is then optimized to minimize dose and particle number.

Formulations can be administered using a metered dose inhaler ("MDI"), a nebulizer, or using a dry powder inhaler. Suitable devices are commercially available and described in the literature.

Aerosol dosage, formulations and delivery systems may be selected for a particular therapeutic application, as described, for example, in Gonda, I. "Aerosols for delivery of therapeutic and diagnostic agents to the respiratory tract," in Critical Reviews in Therapeutic Drug Carrier Systems, 6:273-313, 1990; and in Moren, "Aerosol dosage forms and formulations," in: Aerosols in Medicine, Principles, Diagnosis and Therapy, Moren, et al., Eds. Esevier,Amsterdam, 1985.

Delivery is achieved by one of several methods, for example, using a metered dose inhaler including HFA propellant, a metered dose inhaler with non-HFA propellant, a nebulizer, a pressurized can, or a continuous sprayer. For example, the patient can mix a dried powder of pre-suspended therapeutic with solvent and then nebulize it. It may be more appropriate to use a pre-nebulized solution, regulating the dosage administered and avoiding possible loss of suspension. After nebulization, it may be possible to pressurize the aerosol and have it administered through a metered dose inhaler (MDI). Nebulizers create a fine mist from a solution or suspension, which is inhaled by the patient. The devices described in U.S. Patent No. 5,709,202 to Lloyd, et al., can be used. An MDI typically includes a pressurized canister having a meter valve, wherein the canister is filled with the solution or suspension and a propellant. The solvent itself may function as the propellant, or the composition may be combined with a propellant, such as FREON® (E. I. Du Pont De Nemours and Co. Corp.). The composition is a fine mist when released from the canister due to the release in pressure. The propellant and solvent may wholly or partially evaporate due to the decrease in pressure.

In an alternative embodiment, the formulation is in the form of salt(s) or other conductive material(s) that are dispersed on or in excipient(s). The excipient is preferably a safe (harmless) and biodegradable material. Typical excipients include dextran, lactose, and mannitol.

Individuals to be treated include those at risk of infection, those with a viral or bacterial infection, allergy patients, asthma patients, and individuals working with immunocompromised patients or infected patients.

The formulation may be administered to humans or animals such as racehorses, breeding livestock, or endangered captive animals to protect these animals from infection by viral shedding. This may be accomplished by placing a nebulizer system near watering stations and triggering production of the aerosol as animals either approach or leave the station. Formulation may be sprayed over the animals as they walk through chutes or pens, or sprayed from spray trucks or even crop dusting type airplanes. Individual battery powered sprayers that are currently used to spray insecticides may be adapted for use in administering the solutions to the animals to minimize bioaerosol formation and/or dispersion.

The formulation may be administered to humans or animals at the onset of viral or bacterial outbreak to prevent spread of the disease to epidemic levels. Animals within a 10-kilometer radius of a FMD outbreak are currently deemed infected. These animals are subsequently slaughtered and disinfected. This aerosol system may be administered immediately to animals within this 10-kilometer radius zone and a further prescribed buffer zone outside this area to assure containment of the outbreak. The aerosol can then be administered for as long as is necessary to ensure success, i.e. beyond the normal period between first infection and symptom expression.

The formulation may be administered to humans or animals by creating an aqueous environment in which the humans and animals move or remain for sufficient periods of time to sufficiently treat the lungs. This atmosphere might be created by use of a nebulizer or even a humidifier.

Although described primarily with reference to pulmonary administration, it is understood that the formulations may be administered to individual animals or humans through inhalation; parenteral, oral, rectal, vaginal or topical administration; or by administration to the ocular space.

### IV. Other Methods for Altering Charge in the Mucosal Lining

The alteration of the biophysical properties for the mucosal lining can also be achieved by alternative methods. In one method, electrodes or patches are placed on the body of the individual to be treated and an electric field is generated. This may result in altering charge location, ionic concentration or ionic strength of the mucosal lining and thus modifying its biophysical properties.

The present invention will be further understood by reference to the following non-limiting examples.

### Examples

### Mucus Mimetic Formulation and Methods used *In Vitro* Studies

*Mucus Mimetic Formulation*

Weak polymeric gels with rheological properties similar to tracheobronchial mucus were prepared similar to those described by King et al., Nurs Res. 31(6):324-9 (1982) using locust bean gum (LBG) (Fluka BioChemika) solutions that were crosslinked with sodium tetraborate ($Na_2B_4O_7$ (J.T.Baker). LBG at 2% wt/vol was dissolved in boiling Milli-Q distilled water. A concentrated sodium tetraborate solution was prepared in Milli-Q distilled water. After the LBG solution cooled to room temperature, small amounts of sodium tetraborate solution were added and the mixture was slowly rotated for 1 minute (also referred to herein as "mucus mimetic"). A specific volume of the still watery mucus mimetic was then placed onto a model trachea (a machined trough), creating a mimetic depth based on the trough geometry. The mucus mimetic layers were allowed 15 minutes to crosslink prior to initiation of *the in vitro* experiments. Final concentrations of sodium tetraborate ranged from 1-3 mM.

*Methods*

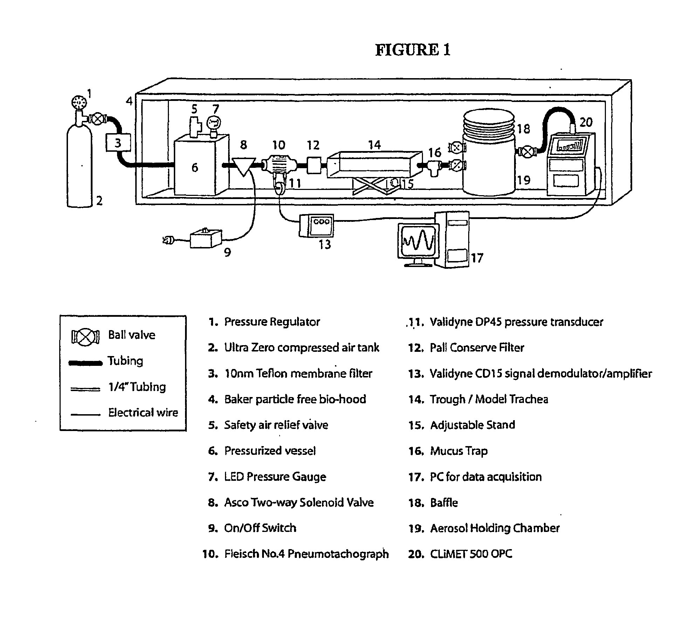

The *following in vitro* method was used to test the effectiveness of different formulations, such as saline or calcium chloride in saline solution. As described above, the mucus mimetic was applied to a model trachea (trough) and allowed 15 minutes to crosslink. The trough was connected to a simulated respiratory machine (a modification of a 'cough' machine by King et al. (M. King, J.M. Zahm, D. Pierrot, S. Vaquez-Girod, E. Puchelle, Biorheology (1989) 26: 737-745)). A respiratory event of prescribed airflow over the surface of the mimetic was initiated through the model trough (this simulated breathing over the mucus layer within the trachea). Figure 1 is a schematic of the simulated respiratory machine that was used in the studies.

Shearing experienced at the mimetic surface caused the formation of aerosol particles which were entrapped in the air-stream and carried downstream. An optical particle counter (OPC) (number 20 in Figure 1) (CLiMET Instruments, Redlands, CA) was placed downstream of the trough to count and measure the size of the aerosol particles generated.

The entire *in vitro* test system is shown in Figure 1. The components are as follows:

1. Compressed air tank - Supplies pressurized air to system
2. Pressure regulator
3. 10nm Teflon membrane filter - Filters compressed air to ensure low particle counts before air enters system
4. Baker bio-hood — Prevents addition of particles from atmosphere
5. Safety air relief valve - Prevents over pressurization of system
6. Air-tight, 6.2L pressurized vessel — Provides controlled release of pressurized air to system (mimics capacitance function of lungs)
7. LED pressure gauge
8. Asco two-way solenoid valve — Electromechanical switch to control delivery of pressurized air to trough
9. Open/closed switch for solenoid valve
10. Fleisch No. 4 Pneumotachograph - Provides laminar air flow to pressure transducer
11. Validyne DP45 pressure transducer - Measures pressure drop through pneumotachograph
12. Pall Conserve filter-Prevents particles generated from mechanical action of solenoid valve from entering system
13. Validyne CD15 signal demodulator/amplifier — Manipulates electrical signal received from pressure transducer for data acquisition system
14. Model trachea (trough) — Machined acrylic 30 cm x 1.6 cm x 1.6 cm (L x W x H) to simulate trachea
15. Adjustable stand — Levels trough
16. Drip trap — Prevents bulk movement of mucus mimetic from entering the holding chamber
17. Computer for data acquisition
18. Baffle — Dampens pressure differences in system to prevent over-pressurization of holding chamber
19. Holding chamber - Prevents escape of aerosol droplets to atmosphere prior to measurement by the OPC
20. CLiMET 500 OPC — Measures number and physical size of aerosol droplets via laser diffraction.

The CLiMET OPC draws an air stream through the path of a laser beam at 1 CFM. Particles within the air stream cause the laser light to diffract when they are struck by the beam, and the intensity of the diffracted light is measured. The intensity and frequency of diffraction are then used to calculate the total number of particles as well as their physical size.

An alternative method can be used which uses labeled nanoparticles that are incorporated into the mimetic, then carried downstream in aerosol droplets and collected from using a filter placed at the exit of the trough for further analysis.

A variety of formulations were tested to determine their effect on the biophysical properties of the mucosal lining, such as surface viscoelasticity and surface tension. Each formulation was introduced onto the mucus layer by aerosolizing the formulation using an appropriate aerosolization method. For solutions/suspensions, an Aeroneb Go nebulizer (Aerogen, Mountain View, CA) was used. The Aeroneb Go nebulizer utilizes vibrating mesh technology to aerosolize the solution. The aerosolization time was set for 2 minutes for all tests. The mimetic was placed at the inlet of the trough and the air pressure was set at 3 psi, mimicking a cough event. These conditions were selected based on the optimized tangent and normal stresses occurring on the bulk mimetic to minimize the movement of the mimetic into the valve/liquid trap and the splashing of the bulk mimetic onto the trough walls.

**Example 1: *In Vitro* Study using the SRM apparatus on the Effect of Different Formulations on Number of Particle Counts**

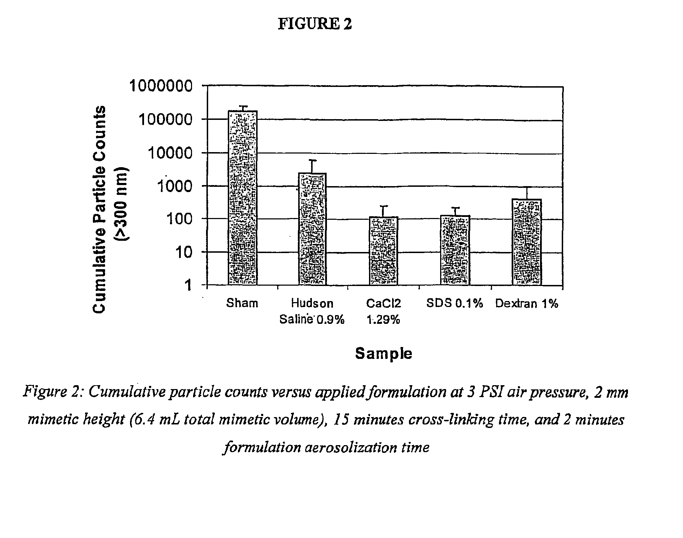

Four formulations were tested *in vitro* using the SRM apparatus described above and compared against the mucus mimetic alone (sham) which was used as a reference. The mucus mimetic production and the SRM method described above were used in each experiment. The following formulations were tested: (1) 0.9% isotonic saline, (2) 1.29% calcium chloride ($CaCl_2$) dissolved in 0.9% isotonic saline solution, (3) 0.1% sodium dodecyl sulfate (SDS) dissolved in 0.9% isotonic saline solution, and (4) 1% dextran dissolved in 0.9% isotonic saline solution. The mimetic height applied onto the trough was maintained at a constant height of 2 mm (6.4 ml total mimetic volume) for all tests. The mimetic was crosslinked for 15 minutes and each formulation was then aerosolized onto the mimetic using the Aeroneb Go (Aerogen, Mountain View, CA) for 2 minutes prior to the test. Each test was repeated at least three times and the average cumulative particle counts and standard deviation values were then calculated. These results are graphically depicted in Figure 2.
As shown in Figure 2, each formulation demonstrated particle suppression of one order of magnitude or greater. The isotonic saline solution containing $CaCl_2$ shows the greatest particle suppression, i.e. three orders of magnitude.

**Example 2: Effect of Formulations (in Saline and Aqueous Solutions) on the Reduction of Exhaled Aerosol Particles as Measured *In Vitro* using the SRM apparatus**

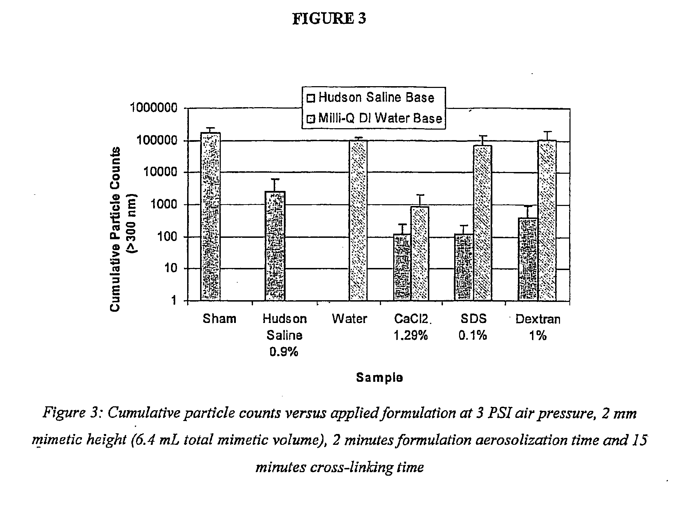

To further understand the mechanism underlying the particle suppression, formulations were prepared both in deionized (DI) water and saline. The mucus mimetic production and the SRM method described above were used in each experiment. The mimetic height applied onto the trough was maintained constant at 2 mm (6.4 ml total mimetic volume) for all tests. The mimetic was crosslinked for 15 minutes and each formulations was then aerosolized onto the mimetic using the Aeroneb Go (Aerogen, Mountain View, CA) for 2 minutes prior to the test. Each test was repeated at least three times and the average cumulative particle counts and standard deviation values were then calculated. The results are graphically depicted in Figure 3.
As shown in Figure 3, when the saline used in a given formulation is replaced with deionized (DI) water, the particle suppression performance of the given formulation decreases. For $CaCl_2$, the formulation's ability to suppress particle formation decreased only slightly when saline was replaced with DI water, and both the DI water and saline formulations containing $CaCl_2$ were better at particle suppression than saline alone. However, for formulations containing SDS or dextran, the amount of suppression becomes negligible when saline is replaced with DI water. These results indicate that the salts (NaCl and $CaCl_2$) play a key roll in the suppression of particle formation.

**Example 3: Conductivity Values of Different Formulations and the Effect of the Formulation Conductivity on the Cumulative Particle Counts as Measured *In Vitro* using the SRM Apparatus**

To determine the effect of the charge/conductivity of a formulation on the suppression of particle formation, the conductivity of different formulations was measured and plotted against the cumulative particle counts. The following ten formulations were tested: (1) saline 0.45%, (2) isotonic saline 0.9%, (3) saline 1.45%, (4) $CaCl_2$ in isotonic saline (1.29%), (5) $CaCl_2$ in DI water (1.29%), (6) $CaCl_2$ in DI water (1.87%), (7) SDS in isotonic saline (0.1 %), (8) SDS in DI water (0.1 %), (9) Dextran in isotonic saline (1%), and (10) Dextran in DI water (1%). The conductivity value for each of the different formulations and for mucus mimetic was measured using the Brookhaven ZetaPALS zetasizer (Brookhaven Instruments, Holtsville, NY). This instrument measures the zetapotential of a given solution/formulation by first measuring its conductivity (to determine the strength of the applied electric field) and then optically measuring the mobility of the solution. The conductivity is reported prior to initiation of the zeta potential measurement, and this value is reported in Table 1. Conductivity is the reciprocal of the electrical resistance of a given sample, and is dependant on the strength/concentration of charge within the sample as well as the mobility of the charge. A geometrical correction factor is applied (called the measurement cell constant, determined by dividing the cell length by the electrode area) which results in the conductivity being reported as the specific conductivity, with units of micro-Siemens per centimeter.

**Table 1: Conductivity of Ten formulations and mucus mimetic**

| Sample | Specific Conductivity ($\mu$S/cm) |
|---|---|
| Mucus | 528 |
| Saline 0.45% | 12,232 |
| Isotonic Saline 0.9% | 23,829 |

(continued)

| Sample | Specific Conductivity ($\mu$S/cm) |
|---|---|
| Saline 1.45% | 33,989 |
| CaCl$_2$ 1.29% (IsotonicSaline) | 37,586 |
| CaCl$_2$ 1.29% (DI Water) | 24,931 |
| CaCl$_2$ 1.87% (DI Water) | 30,187 |
| SDS 0.1% (Isotonic Saline) | 24,045 |
| SDS 0.1 % (DI Water) | 103 |
| Dextran 1 % (Isotonic Saline) | 20,689 |
| Dextran 1% (DI Water) | 60 |

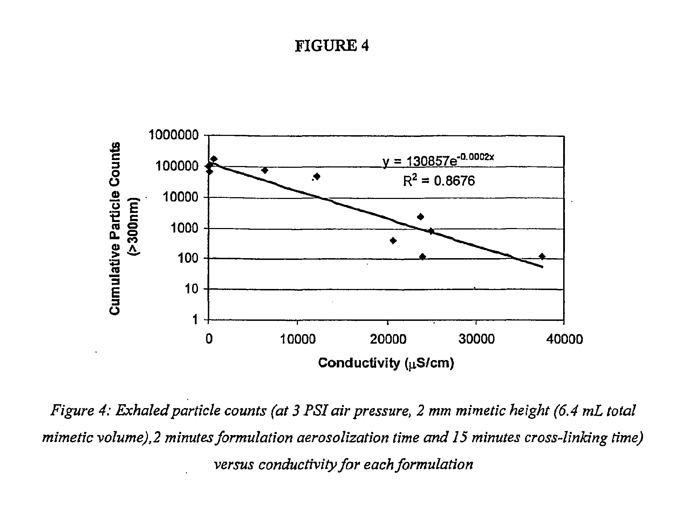

The SRM test and the mucus mimetic production described above were used in this study. The mimetic height applied onto the trough was maintained constant at 2 mm (6.4 ml total mimetic volume) for all tests. The mimetic was crosslinked for 15 minutes and each formulation was then aerosolized onto the mimetic using the Aeroneb Go (Aerogen, Mountain View, CA) for 2 minutes prior to the test. Each test was repeated at least three times and the average cumulative particle counts and standard deviation values were then calculated. Figure 4 is a graph of the conductivity of that formulation versus the exhaled particle counts for that formulation (from *in vitro* SRM tests).

As shown in Figure 4, a strong correlation is evident between the conductivity and the exhaled particle counts, i.e. the higher the conductivity, the lower the total particle count. Thus formulations with higher conductivities result in greater aerosol droplet suppression.

Example 4: Comparison of conductivity values and loss tangent (as measured by an interfacial stress rheometer)

Values for G', G", G*, and Tan $\delta$ were obtained using an interfacial stress rheometer (ISR) for mucus mimetic as well as mimetic with one of several formulations (0.9% NaCl in water (isotonic saline), 1.29% CaCl$_2$ in Saline, 0.1% SDS in Saline, and 1.0% Dextran in Saline) aerosolized onto the surface. The ISR utilized a magnetized rod with a small diameter to length ratio (in order to obtain data at an interface and limit bulk effects). The rod was placed on the surface of the sample which was contained in a small trough. An oscillating magnetic field (with a frequency of 0.25 Hz to simulate breathing) was applied across the sample as the stress, causing the rod to move along the direction of its length. An optical camera captured the movement, and image recognition software was used to calculate the response (the distance the rod moved, or strain). G', G", G*, and the loss tangent (Tan $\delta$) were then determined from this information.

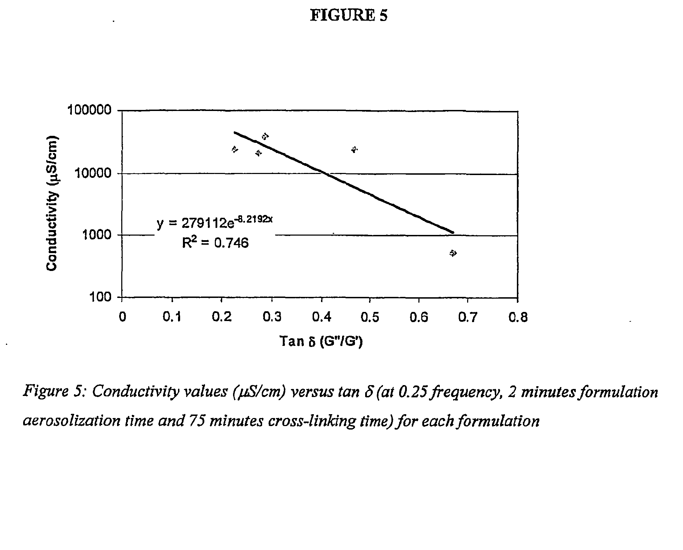

Figure 5 is a graph showing the loss tangent versus the conductivity versus each formulation. A correlation can be seen where the lower the loss tangent (indicating a more elastic sample), the greater the conductivity. When this data is compared with the correlation found between the conductivity and the exhaled particle count (Example 3), it demonstrates a relationship wherein the exhaled particle count decreases with increasing conductivity (increased strength/concentration and mobility of charge) and decreasing loss tangent (increasing elasticity relative to viscosity). This suggests a potential mechanism by which the exhaled particle count is suppressed: the addition of charge through the application of an aerosolized formulation onto the surface of the mimetic alters the viscoelasticity of the mimetic, decreasing the loss tangent and increasing the mechanical rigidity of the surface (G*) through increased cross-linking and chemical bonding at the mimetic surface.

**Example 5: Effectiveness of Different Formulations with Different Conductivity Values on Suppressing Particle Formation when Sheared** at 4 psi

To further distinguish the conductivity/charge effect on particle suppression for different formulations, the pressure used in the SRM testing was increased from 3 to 4 psi. Four formulations, isotonic saline, 1.29% CaCl$_2$ dissolved in isotonic saline, 1.29% CaCl$_2$ dissolved in DI water and 1.8% saline solution, were used in the tests. The conductivity values of the four formulations are tabulated in Table 2. The conductivity value for each of the different formulations was measured using the Brookhaven ZetaPALS zetasizer (Brookhaven Instruments, Holtsville, NY) as described above in Example 3.

**Table 2: Conductivity values of the tested formulations**

| Sample | Specific Conductivity ($\mu$S/cm) |
| --- | --- |
| Saline 0.9% | 23829 |
| Saline 1.8% | 42201 |
| CaCl$_2$ 1.29% (Isotonic Saline) | 37586 |
| CaCl$_2$ 1.29% (DI Water) | 24931 |

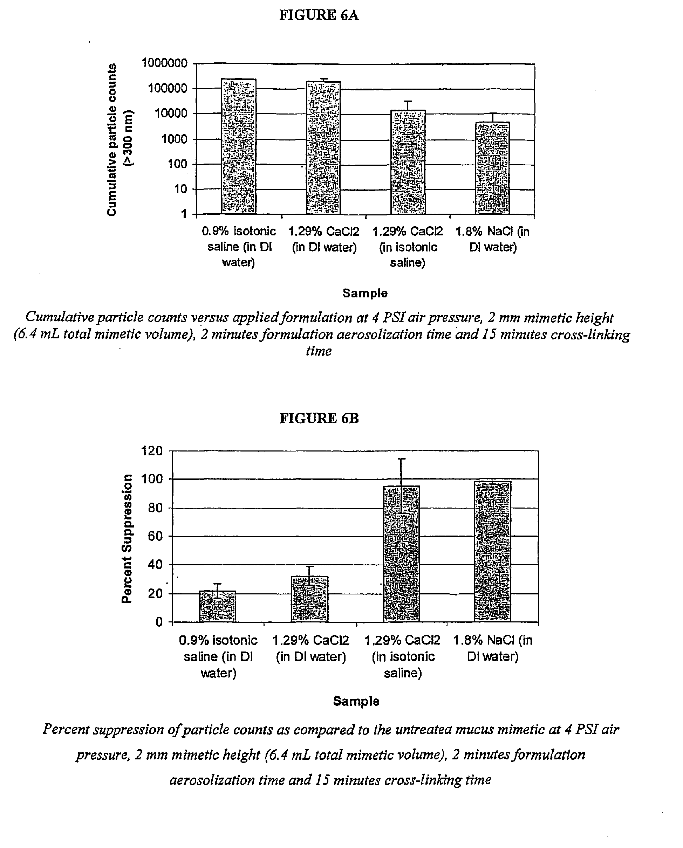

The mucus mimetic production and the SRM method described above were used in each experiment. The mimetic height applied onto the trough was maintained constant at 2 mm (6.4 ml total mimetic volume) for all tests. The mimetic was crosslinked for 15 minutes and each formulations was then aerosolized onto the mimetic using the Aeroneb Go (Aerogen, Mountain View, CA) for 2 minutes prior to the test. Each test was repeated at least three times and the average cumulative particle counts and the percent suppression as compared to the standard mucus mimetic were then calculated and summarized in Figures 6A and 6B, respectively.

As shown in Figures 6A and 6B, the higher the conductivity value of the formulation, the greater its particle suppression capability.

1. A conductive biocompatible formulation comprising a charged compound, wherein the conductive formulation when administered to the mucosal lining fluid or mucosal lining of a human or other animal alters the surface viscoelastic properties of the mucosal lining fluid as defined by the loss tangent (Tan $\delta$), surface tension, or viscosity.

2. The formulation of feature 1 selected from the group consisting of aqueous solutions, dry powders, vapors, aqueous suspensions, non-toxic organic solutions or suspensions, and solid dosage forms other than dry powder.

3. The formulation of feature 1 formulated for administration to a region selected from the group consisting of the respiratory tract, the gastrointestinal tract, the reproductive organs, the ocular region, and the oropharynx or nasal cavities.

4. The formulation feature 1 wherein the charged compound is selected from the group consisting of salts, ionic surfactants, charged amino acids, charged proteins or peptides, charged nucleic acids, and combinations thereof.

5. The formulation feature 1 also including an active agent selected from the group consisting of nucleic acids, proteins, carbohydrates, amino acids, inorganic substances, and organic substances.

6. The formulation feature 4, wherein the charged compound is a salt selected from the group consisting of sodium chloride, sodium acetate, sodium bicarbonate, sodium carbonate, sodium sulfate, sodium stearate, sodium ascorbate, sodium benzoate, sodium biphosphate, sodium phosphate, sodium bisulfite, sodium citrate, sodium borate, calcium chloride, calcium carbonate, calcium acetate, calcium gluconate, calcium phosphate, calcium alginate, calcium stearate, calcium sorbate, calcium sulfate, magnesium carbonate, magnesium sulfate, magnesium stearate, magnesium trisilicate, potassium bicarbonate, potassium chloride, potassium citrate, potassium borate, potassium bisulfite, potassium biphosphate, potassium alginate, potassium benzoate, magnesium chloride, cupric sulfate, chromium chloride, stannous chloride, and sodium metasilicate, and combinations thereof.

7. The formulation feature 4, wherein the charged compound is an ionic surfactant selected from the group consisting of sodium dodecyl sulfate (SDS), magnesium lauryl sulfate, Polysorbate 20, Polysorbate 80, 1,2-dioleoyl-sn-glycero-3-ethylphosphocholine triflate (EDOPC) and alkyl phosphocholine trimesters. .

8. The formulation of feature 1 comprising saline and calcium chloride.

9. The formulation of feature 1, wherein the formulation has a conductivity of greater than 5,000 $\mu$S/cm.

10. The formulation of feature 9, wherein the formulation has a conductivity of greater than 10,000 $\mu$S/cm.

11. The formulation of feature 10, the formulation has a conductivity of greater than 20,000 $\mu$S/cm.

12. The formulation of feature 1, wherein the formulation is capable of altering the viscoelasticity of the mucosal lining so that $\delta$ is greater than 45° and less than 90°.

13. The formulation of feature 1, wherein the formulation is capable of altering the viscoelasticity of the mucosal lining so that $\delta$ is greater than 0° and less than 45°.

14. A method for decreasing exhalation of particles or reducing pathogen intracellular transport in an individual comprising administering to a mucosal lining in the individual an effective amount of a biocompatible formulation comprising a charged compound, wherein the formulation is administered in an effective amount to increase the solidity of the mucosal lining and to modify the viscoelasticity of the mucosal lining so that Tan $\delta$ is less than 1.0.

15. The method of feature 14, wherein the wherein the formulation has a conductivity of greater than 5,000 $\mu$S/cm.

16. A method for increasing drug uptake in an individual, comprising administering to a mucosal lining in the individual an effective amount of a first biocompatible formulation comprising a charged compound, wherein the absorptive and diffusive properties of the mucosal lining and to modify the viscoelasticity of the mucosal lining so that Tan $\delta$ is

greater than 1.0.

17. The method of feature 16, wherein the formulation further comprises an active agent.

18. The method of feature 16, further comprising administering a second formulation to the mucosal lining after the administration of the first formulation, wherein the second formulation comprises an active agent.

19. A method for decreasing or preventing the occurrence of obstructive sleep apnea, irritable bowel syndrome, chronic obstructive pulmonary disease (COPD), cystic fibrosis, or dysentery in an individual comprising administering to a mucosal lining in the individual an effective amount of a biocompatible formulation comprising a charged compound.

20. The method of any one of feature 14-19, wherein the formulation is administered to the respiratory tract in the form of an aerosol.

21. The method of any one of feature 16-18, wherein the first formulation is administered parenterally, orally, rectally, vaginally, topically, or by inhalation.

**Claims**

1.  A calcium salt formulation for use in treating asthma in an individual, the use comprising administering to the individual an effective amount of the calcium salt formulation, wherein asthma is treated.

2.  The calcium salt formulation for use of claim 1, wherein the formulation is administered in the form of an aerosol.

3.  The calcium salt formulation for use of claim 1, wherein said calcium salt formulation is administered to the mucosal lining of the respiratory tract.

4.  The calcium salt formulation for use of claim 1, wherein said calcium salt formulation is administered to the deep lung portion of the respiratory tract.

5.  The calcium salt formulation for use of claim 1, wherein said calcium salt formulation is administered to the upper airways of the respiratory tract.

6.  The calcium salt formulation for use of claim 1, wherein the formulation further comprises a drug; wherein said drug is, optionally, selected from the group consisting of a bronchodilator, an anti-inflammatory, and a steroid.

7.  The calcium salt formulation for use of claim 1, wherein the formulation further comprises a drug; wherein said drug is, optionally, selected from the group consisting of an antiviral, an antibiotic, an antihistamine, a cough suppressant, a mucus production inhibitor, an siRNA, a vaccine, an adjuvant, and an expectorant.

8.  The calcium salt formulation for use of claim 1, wherein said formulation is a dry powder.

9.  The calcium salt formulation for use of claim 1, wherein said formulation is an aqueous solution or suspension.

10. The calcium salt formulation for use of claim 1, wherein said calcium salt formulation is administered by inhalation.

11. The calcium salt formulation for use of any one of claims 1-10, wherein said calcium salt is selected from the group consisting of calcium chloride, calcium carbonate, calcium acetate, calcium gluconate, calcium phosphate, calcium alginate, calcium stearate, calcium sorbate and calcium sulfate; wherein, optionally, said calcium salt is calcium chloride.

12. The calcium salt formulation for use in any one of the preceding claims, wherein said use further comprises administering a second formulation to the mucosal lining after administration of the calcium salt formulation, wherein the second formulation comprises a drug; wherein said drug is, optionally, selected from the group consisting of an antiviral, an antibiotic, an antihistamine, a bronchodilator, a cough suppressant, an anti-inflammatory, a steroid, a mucus production inhibitor, an siRNA, a vaccine, an adjuvant, and an expectorant.

13. The calcium salt formulation for use of any one of the preceding claims, wherein administration of said calcium salt formulation relieves airway irritation and congestion in said individual.

# FIGURE 1

| | |
|---|---|
| 1. Pressure Regulator | 11. Validyne DP45 pressure transducer |
| 2. Ultra Zero compressed air tank | 12. Pall Conserve Filter |
| 3. 10nm Teflon membrane filter | 13. Validyne CD15 signal demodulator/amplifier |
| 4. Baker particle free bio-hood | 14. Trough / Model Trachea |
| 5. Safety air relief valve | 15. Adjustable Stand |
| 6. Pressurized vessel | 16. Mucus Trap |
| 7. LED Pressure Gauge | 17. PC for data acquisition |
| 8. Asco Two-way Solenoid Valve | 18. Baffle |
| 9. On/Off Switch | 19. Aerosol Holding Chamber |
| 10. Fleisch No.4 Pneumotachograph | 20. CLiMET 500 OPC |

Legend:
- ⊗ Ball valve
- ▬ Tubing
- ═ 1/4" Tubing
- — Electrical wire

FIGURE 2

Figure 2: Cumulative particle counts versus applied formulation at 3 PSI air pressure, 2 mm
mimetic height (6.4 mL total mimetic volume), 15 minutes cross-linking time, and 2 minutes
formulation aerosolization time

FIGURE 3

*Figure 3: Cumulative particle counts versus applied formulation at 3 PSI air pressure, 2 mm mimetic height (6.4 mL total mimetic volume), 2 minutes formulation aerosolization time and 15 minutes cross-linking time*

FIGURE 4

*Figure 4: Exhaled particle counts (at 3 PSI air pressure, 2 mm mimetic height (6.4 mL total mimetic volume),2 minutes formulation aerosolization time and 15 minutes cross-linking time) versus conductivity for each formulation*

FIGURE 5

*Figure 5: Conductivity values (µS/cm) versus tan δ (at 0.25 frequency, 2 minutes formulation aerosolization time and 75 minutes cross-linking time) for each formulation*

**FIGURE 6A**

Cumulative particle counts versus applied formulation at 4 PSI air pressure, 2 mm mimetic height (6.4 mL total mimetic volume), 2 minutes formulation aerosolization time and 15 minutes cross-linking time

**FIGURE 6B**

Percent suppression of particle counts as compared to the untreated mucus mimetic at 4 PSI air pressure, 2 mm mimetic height (6.4 mL total mimetic volume), 2 minutes formulation aerosolization time and 15 minutes cross-linking time

EP 2 308 469 A1

**EUROPEAN SEARCH REPORT**

Application Number

EP 10 01 4830

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/004852 A1 (GLAXO GROUP LTD [GB]; THOMAS MARIAN [GB]) 20 January 2005 (2005-01-20) * claims 1-19 * | 1-13 | INV. A61K9/12 A61K47/12 A61K47/02 |
| A | EDWARDS DAVID A ET AL: "Inhaling to mitigate exhaled bioaerosols", PROCEEDINGS OF THE NATIONAL ACADEMY OF SCIENCES OF USA, NATIONAL ACADEMY OF SCIENCE, WASHINGTON, DC, US, vol. 101, no. 50, 14 December 2004 (2004-12-14), pages 17383-17388, XP002392837, ISSN: 0027-8424 * page 17385, column 1, paragraph 2 - page 17386, column 1, paragraph 1 * * page 17387, column 2, paragraph 3 * * figures 1,2 * | 1-13 | |
| A | ROTE LISTE SERVICE: "ROTE LISTE 2002", 2002, EDITIO CANTOR VERLAG, FRANKFURT/MAIN, XP002416908, * paragraph [72087] * * paragraph [28005] * | 1-13 | **TECHNICAL FIELDS SEARCHED (IPC)** A61K |
| A | US 4 637 815 A (LEMOLE GERALD M [US]) 20 January 1987 (1987-01-20) * claim 2 * | 1-13 | |
| A,D | WO 03/092654 A (HARVARD COLLEGE [US]; EDWARDS DAVID A [US]; STONE HOWARD A [US]) 13 November 2003 (2003-11-13) * claims 1,13 * * abstract * | 1-13 | |

The present search report has been drawn up for all claims

| Place of search | Date of completion of the search | Examiner |
|---|---|---|
| Munich | 11 February 2011 | Sindel, Ulrike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

22

## ANNEX TO THE EUROPEAN SEARCH REPORT
## ON EUROPEAN PATENT APPLICATION NO.

EP 10 01 4830

This annex lists the patent family members relating to the patent documents cited in the above-mentioned European search report.
The members are as contained in the European Patent Office EDP file on
The European Patent Office is in no way liable for these particulars which are merely given for the purpose of information.

11-02-2011

| Patent document cited in search report | | Publication date | Patent family member(s) | | Publication date |
|---|---|---|---|---|---|
| WO 2005004852 | A1 | 20-01-2005 | EP | 1646370 A1 | 19-04-2006 |
| | | | JP | 2009514779 T | 09-04-2009 |
| US 4637815 | A | 20-01-1987 | DE | 3671700 D1 | 12-07-1990 |
| | | | EP | 0211629 A1 | 25-02-1987 |
| | | | JP | 1709523 C | 11-11-1992 |
| | | | JP | 3059045 B | 09-09-1991 |
| | | | JP | 62059222 A | 14-03-1987 |
| WO 03092654 | A | 13-11-2003 | AU | 2003243191 A1 | 17-11-2003 |
| | | | CA | 2483917 A1 | 13-11-2003 |
| | | | EP | 1531795 A1 | 25-05-2005 |
| | | | JP | 2005535581 T | 24-11-2005 |
| | | | US | 2009208581 A1 | 20-08-2009 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US SN60682356 P
- WO 03092654 A, David Edwards
- WO 2005084638 A, Pulmatrix
- US 5709202 A, Lloyd

**Non-patent literature cited in the description**

- **FERGUSON et al.** *Nature,* 2001, vol. 414 (6861), 329
- **KIRKNESS, J. P. et al.** *J Physiol,* 2003, vol. 547.2, 603-611
- **WILLIAMS, J. E.** *Alternative Medicine Review,* 2003, vol. 8 (9
- **M. GAD-EL-HAK ; R.F. BLACKWELDER ; J.J. RILEY.** *J. Fluid Mech.,* 1984, vol. 140, 257-280
- **PAPINENI ; ROSENTHAL.** *J. Aerosol Med.,* 1997, vol. 10 (2), 105-116
- **DAWSON, M. et al.** *The Journal of Biological Chemistry,* 2003, vol. 278 (50), 50393-50401
- **ADJEI, A. ; GARREN.** *J. Pharm. Res.,* 1990, vol. 7, 565-569
- **ZANEN, P. ; LAMM, J.-W.** *J.Int. J. Pharm.,* 1995, vol. 114, 111-115
- **J.M. PADFIELD.** Drug Delivery to the Respiratory Tract. Ellis Harwood, 1987
- **N. WORAKUL ; J.R. ROBINSON.** Polymeric Biomaterials. Marcel Dekker, 2002
- **VISSER, J.** *Powder Technology,* 1989, vol. 58, 1-10
- **RUDT, S. ; R. H. MULLER.** *J. Controlled Release,* 1992, vol. 22, 263-272
- **TABATA, Y. ; Y. IKADA.** *J. Biomed. Mater. Res.,* 1988, vol. 22, 837-858
- **GANDERTON, D.** *J. Biopharmaceutical Sciences,* 1992, vol. 3, 101-105
- Physico-Chemical Principles in Aerosol Delivery. **GONDA, I.** Topics in Pharmaceutical Sciences. Medpharm Scientific Publishers, 1991, 95-115
- **FRENCH, D. L. ; EDWARDS, D. A. ; NIVEN, R. W.** *J. Aerosol Sci.,* 1996, vol. 27, 769-783
- **SANDERS, N.N. ; DE SMEDT, S.C. ; ROMPAEY, E.V. ; SIMOENS, P. ; DE BAETS, F. ; DEMEESTER, J.** *Am J Respir Crit Care Med.,* 2000, vol. 162, 1905-1911
- **IM HOF, V. ; GEHR, P. ; GERBER, V. ; LEE, M.M. ; SCHURCH, S.** *Respir. Physiol.,* 1997, vol. 109, 81-93
- **SCHURCH, S. ; GEHR, P. ; IM HOF, V. ; GEISER, M. ; GREEN, F.** *Respir Physiol.,* 1990, vol. 80, 17-32
- **GOLDBERG, S et al.** *J Bacteriology,* 1990, vol. 172, 5650-5654
- **DAVIS, H.E. et al.** *Biophys J,* 2004, vol. 86, 1234-1242
- **J.S. PATTON ; R.M. PLATZ.** *Adv. Drug Del. Rev.,* 1992, vol. 8, 179-196
- **GONDA.** Aerosols for delivery of therapeutic and diagnostic agents to the respiratory tract. *Critical Reviews in Therapeutic Drug Carrier Systems,* 1990, vol. 6, 273-313
- Aerosol dosage forms and formulations. **MOREN et al.** Aerosols in Medicine, Principles, Diagnosis and Therapy. 1985
- **KING et al.** *Nurs Res.,* 1982, vol. 31 (6), 324-9
- **M. KING ; J.M. ZAHM ; D. PIERROT ; S. VAQUEZ-GIROD ; E. PUCHELLE.** *Biorheology,* 1989, vol. 26, 737-745